# EUROPÄISCHE PATENTANMELDUNG

(11) **EP 2 360 149 A1**
(43) Veröffentlichungstag der Anmeldung: **24.08.2011**
(21) Anmeldenummer: 09180969.9
(22) Anmeldetag: 30.12.2009
(51) Int. Cl.: C07D 251/56, C07D 251/70

(54) **Verfahren zur Herstellung von Alkylmelaminen**

(71) Anmelder: Johannes Kepler Universität Linz, 4040 Linz (AT)
(72) Erfinder: List, Manuela, A-5282, Ranshofen (AT); Schwarzinger, Clemens, A-4600, Wels (AT)
(74) Vertreter: Bachinger-Fuchs, Eva-Maria

(57) **Zusammenfassung**

Bei einem Verfahren zur Herstellung von Alkylmelaminen, insbesondere Methylmelaminen, wird N-(C₁-C₄)Alkylharnstoff und/oder N,N'-Di(C₁-C₄)alkylharnstoff eingesetzt und/oder Harnstoff bei einer Temperatur von 300°C-450°C umgesetzt.

## Beschreibung

Die Erfindung betrifft ein Verfahren zur Herstellung von Alkylmelaminen, insbesondere Methylmelaminen.

Aus der DE 680 661 ist bekannt, dass die Synthese von Alkylmelaminen, insbesondere von Methylmelaminen, mittels einer mehrstündigen Reaktion von Melamin mit Methylamin oder Methylaminsalzen unter erhöhter Temperatur ausgeführt werden kann.

Die JP 2000-063369 A beschreibt ein Verfahren zur Alkylierung von Melamin, in dem Melamin mit einem Alkohol bei hohen Temperaturen in Gegenwart eines Metallkatalysators auf einem mikroporösen Träger umgesetzt wird. Dabei werden vor allem niedrig alkylierte Produkte hergestellt. Bei diesem Verfahren wird einerseits eine niedrige Selektivität der alkylierten Produkte und andererseits die Bildung von Cyanursäureestern als Nebenprodukten durch Hydrolyse bzw. Substitution der Aminogruppen beobachtet. Die Reaktion erfolgt unter einer Stickstoff-, Argon-, Wasserstoff oder Kohlenmonoxidatmosphäre.

Gemäß US 2004/0186289 A kann die Synthese von Methylmelaminen mittels einer Reaktion von Melamin mit Methylamin in der Gegenwart von Säure unter erhöhtem Druck durchgeführt werden. Mit diesem Verfahren sind Methylmelamine unterschiedlichen Substitutionsgrads zugänglich, die Reaktion kann allerdings nur chargenweise durchgeführt werden. Darüber hinaus muss bei diesem Verfahren während der Reaktion Ammoniak aus dem Gleichgewicht entfernt werden.

Die EP 0 711 760 A beschreibt die Alkylierung von Melamin durch Umsetzung von Melamin in Gegenwart eines Katalysators und einer Atmosphäre aus Argon, Stickstoff, Kohlenmonoxid oder einer Mischung aus Wasserstoff und Kohlenmonoxid. Es werden aber kein vollständiger Umsatz der Edukte und keine Selektivität bezüglich einzelner Produkte erreicht.

Eine andere Möglichkeit zur Herstellung von Alkylmelaminen ist die Umsetzung von Cyanurchlorid mit Methylamin und/oder Dimethylamin (J. Am. Chem. Soc. 73 (1951), 2984; J. Am. Chem. Soc. 70 (1948), 3726; J. Med. Chem. 22 (1967), 457). Dieser Syntheseweg umfasst allerdings mehrere Verfahrensstufen. Ferner sind der Einsatz von Cyanurchlorid und die Entsorgung der bei der Reaktion anfallenden Salzsäure notwendig. In der EP 1 057 821 A ist die Alkylierung von Melamin mit Alkoholen in Gegenwart von Katalysatoren und einer Stickstoff- oder Wasserstoffatmosphäre beschrieben. Bei diesem Verfahren werden jedoch kein vollständiger Umsatz der Edukte und keine Selektivität bezüglich einzelner Produkte erreicht.

Shinoda et al. (Appl. Catalysis A: General 194-195 (2000), 375-381) beschreiben eine Methylierung von Melamin, ausgehend von Methanol. Für die Katalyse wird ein Metall mit einem sauren Träger benutzt und die Reaktion wird unter Schutzgas- (Argon) oder Wasserstoffatmosphäre durchgeführt. Die Reaktion erreicht zwar einen vollständigen Umsatz, jedoch nur nach sehr langen Reaktionszeiten. Das gebildete Produktspektrum enthält verschieden substituierte Methylmelamine.

Aus der JP 8092226 A ist ein Verfahren zur Herstellung von N-methylierten Melaminderivaten, ausgehend von Melamin-Formaldehyd-Harzen bekannt. Dabei werden die Methylolgruppen des Melaminharzes in einer Wasserstoffatmosphäre mit Hilfe geeigneter Hydrierungskatalysatoren zu Methylgruppen hydriert. Bei diesem Verfahren wird ein hoher Anteil an hochalkylierten N-Methylmelaminen, wie z.B. Hexamethylmelamin, gebildet. Derartige hochalkylierte Verbindungen sind zum einen toxisch und besitzen zum anderen keine am Aminostickstoff gebundenen Wasserstoffatome. Sie sind daher nicht mehr für weitere Syntheseschritte zugänglich.

Die DE 10 2006 005 925 A beschreibt ein Verfahren zur Herstellung von Alkylmelaminen, bei dem Melamin mit einer alkylsubstituierten Ammoniumverbindung, z.B. Tetraalkylammoniumhydroxid, bei Temperaturen zwischen 100°C und 600°C umgesetzt wird.

Gemäß WO 2009/121603 wird Methylmelamin hergestellt, indem Melamin mit Methanol und Ammoniak in Gegenwart eines Metall- oder Metalloxidkatalysators umgesetzt wird.

Die Erfindung stellt sich die Aufgabe, Nachteile und Probleme des Standes der Technik zu überwinden bzw. zu vermeiden und ein einfaches und leicht durchführbares Verfahren zur Herstellung von Alkylmelaminen bereitzustellen, das in bestehenden Melaminanlagen leicht realisierbar ist. Außerdem soll eine Steuerung des Alkylierungsgrades ermöglicht werden.

Diese Aufgabe wird erfindungsgemäß dadurch gelöst, dass N-(C₁-C₄)Alkylharnstoff und/oder N,N'-Di(C₁-C₄)alkylharnstoff und/oder Harnstoff bei einer Temperatur von 300°C-450°C umgesetzt wird. Vorzugsweise werden als N-Alkylharnstoff ein N-(C₁-C₂)Alkylharnstoff und als N,N'-Dialkylharnstoff ein N,N'-Di(C₁-C₂)alkylharnstoff eingesetzt, d.h. die entsprechenden Methyl- und Ethylverbindungen.

Überraschenderweise wurde gefunden, dass es durch den Einsatz von N-Alkylharnstoff und/oder N,N'-Dialkylharnstoff und/oder Harnstoff möglich ist, direkt Alkylmelamine, insbesondere Methylmelamine, zu synthetisieren. Der Alkylierungsgrad kann zudem durch die Wahl der Einsatzprodukte zu einem gewissen Grad gesteuert werden. Beispielsweise wird durch die Verwendung von Harnstoff die Methylierung des Melamins erniedrigt. Das erfindungsgemäße Verfahren eignet sich insbesondere dazu, Trimethylmelamin in guten Ausbeuten mit guten Selektivitäten herzustellen.

Die Umsetzung erfolgt unter Druck, der durch die sich bildenden Gase, hauptsächlich NH₃ und Alkylaminen sowie CO₂ (die ihrerseits wieder zu Harnstoff und Alkylharnstoffen umgesetzt werden können), entsteht. Das erfindungsgemäße Verfahren wird deshalb in Druckbehältern, wie Autoklaven oder Rohrreaktoren, bzw. Anlagen, die den entstehenden Drücken widerstehen können, ausgeführt.

Gemäß einer bevorzugten Ausführungsform des erfindungsgemäßen Verfahrens wird die Umsetzung unter Verwendung eines Katalysators durchgeführt. Hierdurch kann die Reaktion bereits bei geringeren Temperaturen erfolgen. Gleichzeitig wird eine Erhöhung der Umsatzrate und/oder der Selektivität hinsichtlich der Produkte sowie eine Verkürzung der Reaktionszeiten ermöglicht.

Vorzugsweise ist der Katalysator ein dehydratisierender Katalysator, bevorzugt ausgewählt aus der Gruppe, bestehend aus Al₂O₃, Zeolithen, Edelmetall-beladenen Katalysatoren und Mischungen davon, wobei Al₂O₃ besonders bevorzugt ist. Beispiele für Zeolith sind Beta-Zeolith, Y-Zeolith, Faujasit, Mordenit, ZSM-5, Zeolith X oder Zeolith A. Die Zeolithe können auch mit Metallen, z.B. Ru oder Ni, dotiert sein.

Der Katalysator wird bei diskontinuierlichen Verfahren ("Batch") bevorzugt in einer Konzentration von 0,1-2 Gew%, vorzugsweise 0,5-2 Gew%, insbesondere 1-2 Gew%, bezogen auf die Menge der Einsatzstoffe, eingesetzt. Bei kontinuierlichen Verfahren, in denen die Einsatzstoffe zum Beispiel über ein Katalysatorfestbett geführt werden, können die Konzentrationsverhältnisse der Anwendung entsprechend angepasst werden.

Die erforderlichen Reaktionszeiten liegen in einer Ausgestaltung des Verfahrens unter 10 Stunden. Insbesondere wird die katalytische Umsetzung jedoch für eine Dauer von bis zu 5 Stunden durchgeführt, wobei bei kontinuierlichen Verfahren mit Reaktionszeiten von wenigen Sekunden zu rechnen ist.

Eine Ausführungsform des erfindungsgemäßen Verfahrens ist **dadurch gekennzeichnet, dass** die katalytische Umsetzung bei einer Temperatur im Bereich von 320°C-400°C, bevorzugter 320°C-350°C, durchgeführt wird.

In einer bevorzugten Ausgestaltung des erfindungsgemäßen Verfahrens wird die katalytische Umsetzung in Anwesenheit von einer oder mehreren Verbindungen aus der Gruppe von Methanol, Ethanol, Methylamin, Ethylamin, Dimethylamin, Diethylamin und Ammoniak durchgeführt. Durch den Einsatz dieser Stoffe lässt sich eine weitere Selektion und Steuerung der Ausgangsprodukte erzielen.

Wird dem Reaktionsgemisch NH₃ zugegeben, so werden an den Triazinring Aminogruppen gebunden, d.h. der Methylierungsgrad wird erniedrigt. Außerdem verhindert die Zugabe von NH₃ die Bildung von Trimethylisocyanurat, welches durch die Gegenwart von sauerstoffhaltigen Verbindungen entsteht. Bei Zugabe von Methylamin oder Dimethylamin werden Methyl- bzw. Dimethylgruppen (= 2 Methylgruppen an einem Stickstoff des Melamins) eingebaut und der Methylierungsgrad des Melamins erhöht. Analoges gilt für Ethylamin und Diethylamin. Durch den Einsatz von Alkoholen, wie Methanol und Ethanol, werden die Alkylgruppen *in situ* auf das Melamin "aufgebracht".

Die Erfindung wird nachfolgend anhand von Beispielen näher erläutert.
Beispiel 1:
   3 g N-Methylharnstoff werden mit 0,1 Gew% Al₂O₃ gemischt und in einen Autoklaven überführt. Die Mischung wird für 1 Stunde auf 350°C erwärmt und danach abgekühlt. Der Inhalt des Autoklaven wird mit einer Mischung aus Aceton/Wasser im Verhältnis 1:1 herausgewaschen und der Katalysator durch Zentrifugation abgetrennt. Die verbleibende Lösung wird im Vakuum zur Trockene eingedampft und das Produkt mittels GC/MS und GC/WLD charakterisiert.
      Ausbeute: 1,4 g
      Zusammensetzung: 68% Dimethylmelamin; 30% Trimethylmelamin; Trimethylisocyanurat
Beispiel 2:
   3 g N,N'-Dimethylharnstoff werden mit 0,1 Gew% Al₂O₃ gemischt und in einen Autoklaven überführt. Die Mischung wird für 1 Stunde auf 350°C erwärmt und danach abgekühlt. Der Inhalt des Autoklaven wird mit einer Mischung aus Aceton/Wasser im Verhältnis 1:1 herausgewaschen und der Katalysator durch Zentrifugation abgetrennt. Die verbleibende Lösung wird im Vakuum zur Trockene eingedampft und das Produkt mittels GC/MS und GC/WLD charakterisiert.
      Ausbeute: 1,7 g
      Zusammensetzung: 10% Dimethylmelamin; 15% Trimethylmelamin; 1% Tetramethylmelamin; Trimethylisocyanurat
Beispiel 3:
   3 g N-Methylharnstoff werden mit 0,1 Gew% Al₂O₃ gemischt, in einen Autoklaven überführt und 0,7 g Ammoniak zudosiert. Die Mischung wird für 2 Stunden auf 350°C erwärmt und danach abgekühlt. Der Inhalt des Autoklaven wird mit einer Mischung aus Aceton/Wasser im Verhältnis 1:1 herausgewaschen und der Katalysator durch Zentrifugation abgetrennt. Die verbleibende Lösung wird im Vakuum zur Trockene eingedampft und das Produkt mittels GC/MS und GC/WLD charakterisiert.
      Ausbeute: 1,4 g
      Zusammensetzung: 74% Dimethylmelamin; 25% Trimethylmelamin
Beispiel 4:
   3 g N-Methylharnstoff werden mit 0,1 Gew% Al₂O₃ gemischt, in einen Autoklaven überführt und 8 g Ammoniak zudosiert. Die Mischung wird für 2 Stunden auf 350°C erwärmt und danach abgekühlt. Der Inhalt des Autoklaven wird mit einer Mischung aus Aceton/Wasser im Verhältnis 1:1 herausgewaschen und der Katalysator durch Zentrifugation abgetrennt. Die verbleibende Lösung wird im Vakuum zur Trockene eingedampft und das Produkt mittels GC/MS und GC/WLD charakterisiert.
      Ausbeute: 1,5 g
      Zusammensetzung: 89% Dimethylmelamin; 11 % Trimethylmelamin
Beispiel 5:
   1,5 g N-Methylharnstoff und 1,5 g Harnstoff werden mit 0,1 Gew% Al₂O₃ gemischt und in einen Autoklaven überführt. Die Mischung wird für 1 Stunden auf 350°C erwärmt und danach abgekühlt. Der Inhalt des Autoklaven wird mit einer Mischung aus Aceton/Wasser im Verhältnis 1:1 herausgewaschen und der Katalysator durch Zentrifugation abgetrennt. Die verbleibende Lösung wird im Vakuum zur Trockene eingedampft und das Produkt mittels GC/MS und GC/WLD charakterisiert.
      Ausbeute: 0,9 g
      Zusammensetzung: 25% Melamin; 38% Methylmelamin; 25% Dimethylmelamin; 8% Trimethylmelamin
Beispiel 6:
   1,5 g N,N'-Dimethylharnstoffund 1,5 g Harnstoff werden mit 0,1 Gew% Al₂O₃ gemischt und in einen Autoklaven überführt. Die Mischung wird für 1 Stunden auf 350°C erwärmt und danach abgekühlt. Der Inhalt des Autoklaven wird mit einer Mischung aus Aceton/Wasser im Verhältnis 1:1 herausgewaschen und der Katalysator durch Zentrifugation abgetrennt. Die verbleibende Lösung wird im Vakuum zur Trockene eingedampft und das Produkt mittels GC/MS und GC/WLD charakterisiert.
      Ausbeute: 1 g
      Zusammensetzung: 7% Methylmelamin; 38% Dimethylmelamin; 47% Trimethylmelamin; 4% Tetramethylmelamin
Beispiel 7:
   3 g N,N'-Dimethylharnstoff werden mit 0,1 Gew% Al₂O₃ gemischt, in einen Autoklaven überführt und 1,5 g Ammoniak zudosiert. Die Mischung wird für 2 Stunden auf 350°C erwärmt und danach abgekühlt. Der Inhalt des Autoklaven wird mit einer Mischung aus Aceton/Wasser im Verhältnis 1:1 herausgewaschen und der Katalysator durch Zentrifugation abgetrennt. Die verbleibende Lösung wird im Vakuum zur Trockene eingedampft und das Produkt mittels GC/MS und GC/WLD charakterisiert.
      Ausbeute: 1,1 g
      Zusammensetzung: 60% Dimethylmelamin; 30% Trimethylmelamin
Beispiel 8:
   3 g Harnstoff werden mit 0,1 Gew% Al₂O₃ gemischt, in einen Autoklaven überführt und 1,5 g Methylamin zudosiert. Die Mischung wird für 2 Stunden auf 350°C erwärmt und danach abgekühlt. Der Inhalt des Autoklaven wird mit einer Mischung aus Aceton/Wasser im Verhältnis 1:1 herausgewaschen und der Katalysator durch Zentrifugation abgetrennt. Die verbleibende Lösung wird im Vakuum zur Trockene eingedampft und das Produkt mittels GC/MS und GC/WLD charakterisiert.
      Ausbeute: 1,4 g
      Zusammensetzung: 10% Methylmelamin; 38% Dimethylmelamin; 36% Trimethylmelamin
Beispiel 9:
   3 g Harnstoff werden mit 1 g Methanol, 0,1 Gew% Al₂O₃ und 0,1 Gew% eines Ruthenium dotierten Zeolith gemischt, in einen Autoklaven überführt und 5 g Ammoniak zudosiert. Die Mischung wird für 2 Stunden auf 350°C erwärmt und danach abgekühlt. Der Inhalt des Autoklaven wird mit einer Mischung aus Aceton/Wasser im Verhältnis 1:1 herausgewaschen und der Katalysator durch Zentrifugation abgetrennt. Die verbleibende Lösung wird im Vakuum zur Trockene eingedampft und das Produkt mittels GC/MS und GC/WLD charakterisiert.
      Ausbeute: 1,1 g
      Zusammensetzung: 15% Melamin; 28% Methylmelamin; 35% Dimethylmelamin; 18% Trimethylmelamin; 3% Tetramethylmelamin
Beispiel 10:
   3 g N-Methylhamstoff werden in einen Autoklaven überführt und die Mischung für 1 Stunde auf 420°C erwärmt und danach abgekühlt. Der Inhalt des Autoklaven wird mit einer Mischung aus Aceton/Wasser im Verhältnis 1:1 herausgewaschen und der Katalysator durch Zentrifugation abgetrennt. Die verbleibende Lösung wird im Vakuum zur Trockene eingedampft und das Produkt mittels GC/MS und GC/WLD charakterisiert.
      Ausbeute: 1,3 g
      Zusammensetzung: 5% Methylmelamin; 61% Dimethylmelamin; 22% Trimethylmelamin; Trimethylisocyanurat
Beispiel 11:
   3 g Harnstoff werden mit 3 g Ethanol, 0,1 Gew% Al₂O₃ und 0,1 Gew% eines Nickel dotierten Zeolith gemischt, in einen Autoklaven überführt und 5 g Ammoniak zudosiert. Die Mischung wird für 2 Stunden auf 370°C erwärmt und danach abgekühlt. Der Inhalt des Autoklaven wird mit einer Mischung aus Aceton/Wasser im Verhältnis 1:1 herausgewaschen und der Katalysator durch Zentrifugation abgetrennt. Die verbleibende Lösung wird im Vakuum zur Trockene eingedampft und das Produkt mittels GC/MS und GC/WLD charakterisiert.
      Ausbeute: 1,3 g
      Zusammensetzung: 12% Melamin; 27% Ethylmelamin; 40% Diethylmelamin; 13% Triethylmelamin; 5% Tetraethylmelamin
Beispiel 12:
   3 g N-Ethylharnstoff werden mit 0,1 Gew% Al₂O₃ gemischt und in einen Autoklaven überfiihrt. Die Mischung wird für 1 Stunde auf 350°C erwärmt und danach abgekühlt. Der Inhalt des Autoklaven wird mit einer Mischung aus Aceton/Wasser im Verhältnis 1:1 herausgewaschen und der Katalysator durch Zentrifugation abgetrennt. Die verbleibende Lösung wird im Vakuum zur Trockene eingedampft und das Produkt mittels GC/MS charakterisiert.
      Ausbeute: 1,7 g
      Zusammensetzung: 45% Diethylmelamin; 51% Triethylmelamin; Triethylisocyanurat

## Patentansprüche

1. Verfahren zur Herstellung von Alkylmelaminen, insbesondere Methylmelaminen, **dadurch gekennzeichnet, dass** N-(C₁-C₄)Alkylharnstoff und/oder N,N'-Di(C₁-C₄)alkylharnstoff und/oder Harnstoff bei einer Temperatur von 300°C-450°C umgesetzt wird.

2. Verfahren nach Anspruch 1, **dadurch gekennzeichnet, dass** als N-Alkylharnstoff N-(C₁-C₂)Alkylharnstoffund als N,N'-Dialkylharnstoff N,N'-Di(C₁-C₂)alkylharnstoff eingesetzt wird.

3. Verfahren nach Anspruch 1 oder 2, **dadurch gekennzeichnet, dass** die Umsetzung unter Verwendung eines Katalysators, vorzugsweise eines dehydratisierenden Katalysators, durchgeführt wird.

4. Verfahren nach Anspruch 3, **dadurch gekennzeichnet, dass** der Katalysator ausgewählt ist aus der Gruppe, bestehend aus Al₂O₃, Zeolithen, Edelmetall-beladenen Katalysatoren und Mischungen davon.

5. Verfahren nach einem der Ansprüche 1 bis 4, **dadurch gekennzeichnet, dass** der Katalysator in einer Konzentration von 0,1-2 Gew%, vorzugsweise 0,5-2 Gew%, insbesondere 1-2 Gew%, bezogen auf die Menge der Einsatzstoffe, eingesetzt wird.

6. Verfahren nach einem der Ansprüche 1 bis 5, **dadurch gekennzeichnet, dass** die katalytische Umsetzung für eine Dauer von bis zu 5 Stunden durchgeführt wird.

7. Verfahren nach einem der Ansprüche 1 bis 6, **dadurch gekennzeichnet, dass** die katalytische Umsetzung bei einer Temperatur im Bereich von 320°C-400°C, bevorzugter 320°C-350°C, durchgeführt wird.

8. Verfahren nach einem der Ansprüche 1 bis 6, **dadurch gekennzeichnet, dass** die katalytische Umsetzung in Anwesenheit von einer oder mehreren Verbindungen aus der Gruppe von Methanol, Ethanol, Methylamin, Ethylamin, Dimethylamin, Diethylamin und Ammoniak durchgeführt wird.
